# DEMANDE DE BREVET EUROPEEN

(11) **EP 0 643 047 A1**
(43) Date de publication de la demande: **15.03.1995**
(21) Numéro de dépôt: 94401963.7
(22) Date de dépôt: 05.09.1994
(51) Int. Cl.: C07D 233/54, C07D 401/06, A61K 31/445

(54) **Dérivés de 1- 2-amino-5- 1-(triphénylméthyl)-1H-imidazol-4-yl -1-oxopentyl pipéridine, leur préparation et leur utilisation comme intermédiaires de synthèse**

(30) Priorité: 14.09.1993 FR 9310906
(71) Demandeur: SYNTHELABO, F-92350 Le Plessis Robinson (FR)
(72) Inventeur: Lassalle, Gilbert, F-92140 Clamart (FR); Galtier, Daniel, F-78280 Guyancourt (FR); Galli, Frédéric, F-78170 La Celle St Cloud (FR)
(74) Mandataire: Thouret-Lemaitre, Elisabeth

(57) **Abrégé**

Composés répondant à la formule (I) 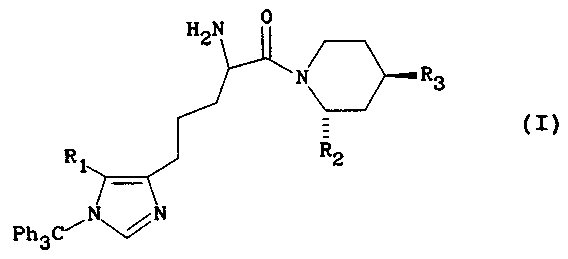
dans laquelle
R₁ représente soit un atome d'hydrogène, soit un groupe (C₁-C₄) alkyle,
R₂ représente soit un atome d'hydrogène, soit un groupe (C₁-C₄)alcoxycarbonyle, soit un groupe carboxylique, soit un groupe carboxylate de sodium, soit un groupe -CH₂OR₄ où R₄ est un atome d'hydrogène, un groupe (C₁-C₄)alkyle ou (C₁-C₄)acyle, soit un groupe -CONR₅R₆, soit un groupe -CN₄R₅ où R₅ est un atome d'hydrogène ou un groupe (C₁-C₄)alkyle et R₆ est un atome d'hydrogène, un groupe (C₁-C₄)alkyle ou hydroxy ou (C₁-C₄)alcoxy ou (C₁-C₃)alcoxyphényle et
R₃ représente un groupe (C₁-C₄)alkyle,
ainsi que leurs sels d'addition aux acides.

Intermédiaires de synthèse.

## Description

La présente invention a pour objet des dérivés de 1-[2-amino-5-[1-(triphénylméthyl)-1*H*-imidazol-4-yl]-1-oxopentyl]pipéridine, leur préparation et leur utilisation comme intermédiaires de synthèse.

Les composés de l'invention répondent à la formule (I) 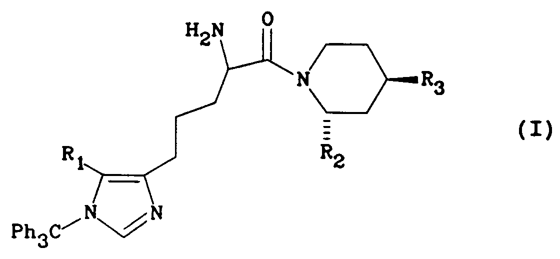
dans laquelle
R₁ représente soit un atome d'hydrogène, soit un groupe (C₁-C₄) alkyle,
R₂ représente soit un atome d'hydrogène, soit un groupe (C₁-C₄)alcoxycarbonyle, soit un groupe carboxylique, soit un groupe carboxylate de sodium, soit un groupe -CH₂OR₄ où R₄ est un atome d'hydrogène, un groupe (C₁-C₄)alkyle ou (C₁-C₄)acyle, soit un groupe -CONR₅R₆, soit un groupe -CN₄R₅ où R₅ est un atome d'hydrogène ou un groupe (C₁-C₄)alkyle et R₆ est un atome d'hydrogène, un groupe (C₁-C₄)alkyle ou hydroxy ou (C₁-C₄)alcoxy ou (C₁-C₃)alcoxyphényle et
R₃ représente un groupe (C₁-C₄)alkyle.

Les composés préférés selon l'invention sont ceux répondant à la formule (I) dans laquelle
R₁ représente soit un atome d'hydrogène, soit un groupe (C₁-C₄) alkyle,
R₂ représente soit un groupe carboxylique, soit un groupe éthoxycarbonyle, soit un groupe hydroxycarboxamide, soit un groupe hydroxyméthyle, soit un groupe 1*H*-tétrazolyle et
R₃ représente un groupe méthyle ou éthyle.

Les composés de l'invention possèdent 3 centres asymétriques selon la signification des substituants.

La configuration préférentielle du groupe pipéridinyle est [2R,4R].
La configuration préférentielle du carbone asymétrique de la partie acide aminé centrale 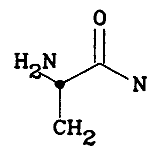
est [S].

Les composés de l'invention peuvent exister sous forme de bases libres ou de sels d'addition aux acides.

Les composés de formule (I) sont utiles pour la synthèse de composés à activité antithrombotique tels que ceux décrits dans la demande de brevet européen EP 0565396 de la demanderesse.

Les composés selon l'invention peuvent être synthétisés selon le schéma 1 de la page suivante.
On fait réagir un alcool de formule (II) avec du chlorure de thionyle dans un mélange de diméthylformamide/dichlorométhane puis on fait réagir le composé obtenu avec de la triphénylphosphine dans un solvant tel que le diméthylformamide ou le benzène à une température de 80°C; on obtient un chlorure de triphénylméthylphosphonium de formule (III) que l'on fait réagir avec un composé de formule (IV); la réaction est conduite dans un solvant tel que le tétrahydrofurane, en présence de n-butyllithium à une température de -70°C. On obtient un composé de formule (V), sous forme d'un mélange cis/trans au niveau de la double liaison. Ensuite on fait réagir le composé de formule (V) avec de l'acide chlorhydrique gazeux dans un solvant tel que le benzène et on obtient un composé de formule (VI) sur lequel on fait réagir un composé de formule (VII) en présence d'une base telle que 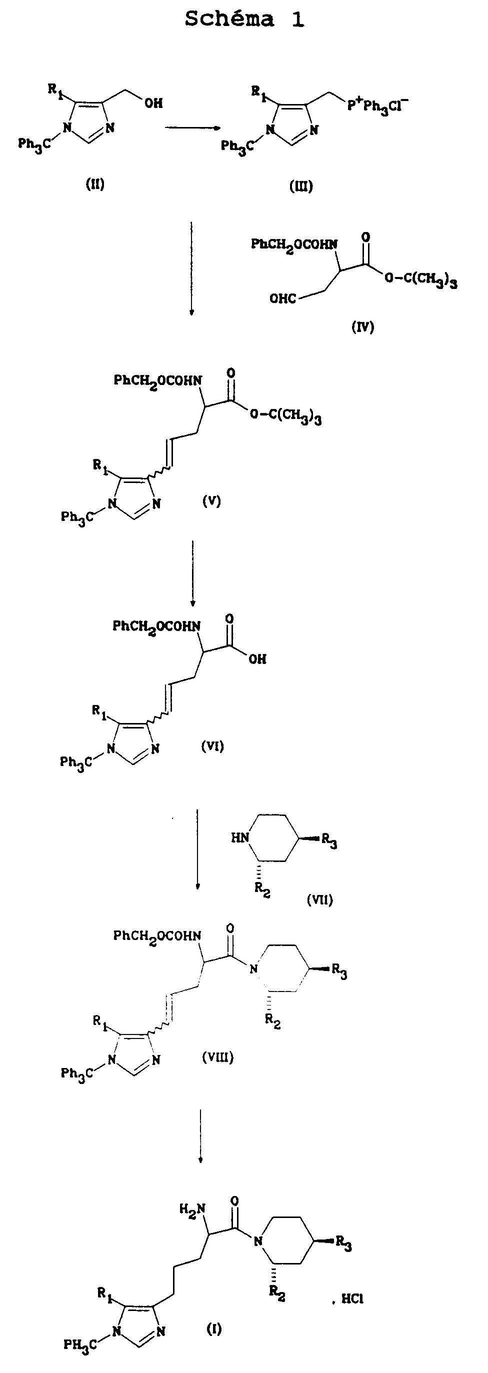
la N,N-diisopropyléthylamine et d'un agent couplant comme l'hexafluorophosphate de [(benzotriazol-1-yl)oxy]tris(diméthylamino)phosphonium pour obtenir un composé de formule (VIII). Puis on soumet le composé de formule (VIII) à une hyrogénation catalytique pour obtenir un composé de formule (I).

Les composés de départ sont disponibles dans le commerce ou décrits dans la littérature ou peuvent être préparés selon des méthodes qui y sont décrites ou qui sont connues de l'homme du métier.
Ainsi les composés de formule (II) sont préparés par des méthodes analogues à celles décrites dans le brevet européen 0242973 et à celles décrites par Griffith R.K. et coll., Synthesis, 1983, 576.
Certains composés de formule (IV) sont décrits par Valerio R.M. et coll. dans Synthesis, 1988, 786.
La préparation des pipéridines de formule (VII) dans laquelle R₂ représente un groupe (C₁-C₄)alcoxycarbonyle ou un groupe carboxylique est décrite dans le brevet européen no 0008746. La préparation des autres pipéridines de formule (VII) est décrite dans la demande de brevet européen EP 0562396 de la demanderesse.
La préparation des composés de formule (V) est décrite dans la demande de brevet européen EP 0562396 de la demanderesse.

Les exemples suivants illustrent la préparation de certains composés conformément à l'invention.
Les microanalyses et les spectres IR et RMN confirment la structure des composés obtenus.

### Exemple 1

chlorhydrate de l'acétate de [2*R*-[1(2*S*), 2α, 4β]]-[1-[2-amino-1-oxo-5-[1-(triphénylméthyl)-1*H*-imidazol-4-yl]pentyl]-4-méthylpipéridin-2-yl]méthyle
1.1. chlorure de triphényl[[(1-triphénylméthyl)-1*H*-imidazol-4-yl]méthyl]phosphonium
   A 670 ml d'une solution de 105,5 g (294 mmoles) de 4-(chlorométhyl)-1-(triphénylméthyl)-1*H*-imidazole dans le diméthylformamide, on ajoute 77,7 g (296 mmoles) de triphénylphosphine. On chauffe à 80 °C pendant 3 heures. On évapore le solvant, on reprend le brut dans l'éther et on le triture. On filtre le précipité et on le sèche sous vide sur pentoxyde de phosphore.
   On obtient 162 g de produit sous forme de cristaux jaunâtres.
   Point de fusion = 210 °C Rendement = 89 %
1.2. (*S*,*E*)-2-[[(phénylméthoxy)carbonyl]amino]-5-[1-(triphénylméthyl)-1*H*-imidazol-4-yl]pent-4-énoate de 1,1-diméthyléthyle
   Dans un ballon tricol, sous argon, on introduit 50,93 g (820 mmoles) de chlorure de triphényl[[1-triphénylméthyl)-1*H*-imidazol-4-yl]méthyl]phosphonium en solution dans 333 ml de tétrahydrofurane. On ajoute goutte à goutte, à - 70 °C, 51,2 ml d'une solution 1,6 M de n-butyllithium dans l'hexane (820 mmoles). Aprés 30 minutes d'agitation à - 70 °C, on verse rapidement le milieu réactionnel dans 270 ml d'une solution 0,253 M, refroidie à -70 °C, de (*S*)-4-oxo-2-[[(phénylméthoxy)carbonyl]amino]butanoate de 1,1-diméthyléthyle dans le tétrahydrofurane (683 mmoles). On laisse remonter le mélange à la température ambiante pendant une nuit. On hydrolyse le mélange avec 280 ml d'une solution aqueuse saturée de chlorure de sodium. On sépare la phase aqueuse de la phase organique et on l'extrait par 2 fois 140 ml d'acétate d'éthyle. On réunit les phases organiques, on les sèche sur du sulfate de magnésium et on évapore à sec. On purifie par chromatographie sur colonne de gel de silice en éluant par un gradient hexane/acétate d'éthyle.
   On obtient un mélange d'oléfine cis et trans.
   Pour la forme cis:
   Point de fusion = 66 °C
   R_{f} = 0,30 [hexane/acétate d'éthyle (60/40)]
   Pour la forme trans:
   R_{f} = 0,15 [hexane/acétate d'éthyle (60/40)]
   Rendement = 40 %
1.3. acide (*S*,*E*)-2-[[(phénylméthoxy)carbonyl]amino]-5-[1-(triphénylméthyl)-1*H*-imidazol-4-yl]pent-4-ènoïque
   On place 3,9 g (6,37 mmoles) du composé trans obtenu dans l'étape précédente dans 80 ml de benzène puis on fait passer à 0 °C un courant d'acide chlorhydrique gazeux jusqu'à saturation. Ensuite on laisse sous agitation pendant 4 heures à la température ambiante puis on évapore à sec. On reprend le résidu par 20 ml de dichlorométhane, on le neutralise par de l'ammoniac et on le purifie par chromatographie sur colonne de gel de silice en éluant par un mélange dichlorométhane/méthanol (90:10).
   On obtient 3 g de produit.
   Point de fusion = 180 °C (décomposition)
1.4. [2*R*-[1(1*S*), 2α, 4β]]-[1-[[2-(acétyloxy)méthyl]-4-méthyl-pipéridin-1-yl]carbonyl]-4-[1-(triphénylméthyl)-1*H*-imidazol-4-yl]but-3-ényl]carbamate de phénylméthyle
   A une solution de 1,14 g (3 mmoles) de trifluoroacétate d'acétate de (2*R*-trans)-(4-méthylpipéridin-2-yl)méthyle (2:1) dans 20 ml de dichlorométhane, on ajoute à 0 °C, 1,67 g (3 mmoles) du composé obtenu dans l'étape précédente, 1,5 ml de N,N-diisopropyléthylamine puis 1,5 g (3,3 mmoles) d'hexafluorophosphate de [(benzotriazol-1-yl)oxy]tris(diméthylamino)phosphonium. On laisse le mélange pendant une nuit à la température ambiante puis on le verse sur 100 ml d'acétate d'éthyle. On lave successivement par 100 ml d'une solution 0,1 N d'acide chlorhydrique, 100 ml d'une solution saturée d'hydrogénocarbonate de sodium, 100 ml d'une solution saturée de chlorure de sodium puis on sèche sur sulfate de magnésium et on évapore à sec.
   On obtient 1,4 g de produit sous forme d'un solide.
   Point de fusion = 59 °C
1.5. chlorhydrate de l'acétate de [2*R*-[1(2*S*), 2α, 4β]]-[1-[2-amino-1-oxo-5-[1-(triphénylméthyl)-1*H*-imidazol-4-yl]pentyl]-4-méthylpipéridin-2-yl]méthyle
   Dans une fiole de Parr, on place 1,3 g (1,8 mmoles) du composé obtenu dans l'étape précédente et on ajoute 30 ml d'éthanol et 0,4 g de palladium sur charbon à 10 %. On hydrogène à 50 psi pendant 8 heures puis on filtre et on essore le catalyseur. On recueille le filtrat et on l'évapore à sec. On reprend le résidu dans 20 ml d'isopropanol chlorhydrique 0,1 N puis on évapore à sec.
   On obtient 1,12 g de produit sous forme de chlorhydrate.
   R_{f} = 0,55 [méthylisobutylcétone/acide acétique/eau (60/20/20)]

### Exemple 2

chlorhydrate de [2*R*-[1(2*S*), 2α, 4β]]-1-[2-amino-1-oxo-5-[1-(triphénylméthyl)-1*H*-imidazol-4-yl]pentyl]-4-méthylpipéridine-2-carboxylate d'éthyle
2.1. [2*R*-[1(2*S*), 2α, 4β]]-4-méthyl-1-[1-oxo-2-[[(phénylméthoxy)carbonyl]amino]-5-[1-(triphénylméthyl)-1*H*-imidazol-4-yl]pent-4-ényl]pipéridine-2-carboxylate d'éthyle
   A une solution de 1,78 g (5 mmoles) de trifluoroacétate de (2*R*-*trans*)-(4-méthylpipéridin-2-yl)carboxylate d'éthyle dans 20 ml de dichlorométhane, on ajoute à 0 °C, 2,9 g (5 mmoles) du composé obtenu selon la méthode décrite dans l'exemple 1.3, 2,5 ml de N,N-diisopropyléthylamine puis 2,3 g (5,2 mmoles) d'hexafluorophosphate de [(benzotriazol-1-yl)oxy]tris(diméthylamino)phosphonium. On laisse le mélange pendant une nuit à la température ambiante puis on le verse sur 100 ml d'acétate d'éthyle. On lave successivement par 100 ml d'une solution 0,1 N d'acide chlorhydrique, 100 ml d'une solution saturée d'hydrogénocarbonate de sodium, 100 ml d'une solution saturée de chlorure de sodium puis on sèche sur sulfate de magnésium et on évapore à sec.
   On obtient 2,4 g de produit sous forme d'un solide.
   Point de fusion = 60-65 °C
2.2. chlorhydrate de [2*R*-[1(2*S*), 2α, 4β]]-1-[2-amino-1-oxo-5-[1-(triphénylméthyl)-1*H*-imidazol-4-yl]pentyl]-4-méthyl-pipéridine-2-carboxylate d'éthyle
   Dans une fiole de Parr, on place 1,65 g (2,3 mmoles) du composé obtenu dans l'étape précédente, 30 ml d'éthanol et 0,66 g de palladium sur charbon à 10 %. On hydrogène à 50 psi pendant 4 heures à froid puis on filtre et on essore le catalyseur. On recueille le filtrat et on l'évapore à sec sous pression réduite. On reprend le résidu dans 23 ml d'isopropanol chlorhydrique 0,1 N puis on évapore à sec.
   On obtient 1,5 g de produit sous forme de chlorhydrate.
   Point de fusion = 98 °C
   Les composés de l'invention sont utiles pour la synthèse de composés de formule (1)

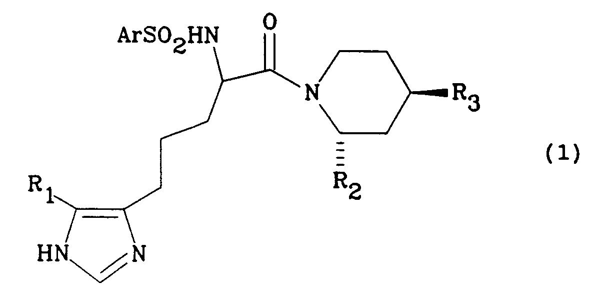

dans laquelle
R₁, R₂ et R₃ sont tels que définis précédemment et
Ar représente soit un groupe naphtalén-1-yle substitué par un groupe di(C₁-C₄)alkylamino, soit un groupe 6,7-di(C₁-C₄)alcoxy naphtalén-1-yle, soit un groupe quinoléin-8-yle substitué en 3 par un groupe (C₁-C₄)alkyle, soit un groupe 1,2,3,4-tétrahydroquinoléin-8-yle substitué en 3 par un groupe (C₁-C₄)alkyle, soit un groupe 1*H*-indazol-7-yle.
La synthèse des composés de formule (1) à partir des composés selon l'invention est décrite dans le schéma 2.
On fait réagir un composé de formule (I) dans laquelle R₁, R₂ et R₃ sont tels que définis précédemment avec un composé de formule ArSO2Cl dans laquelle Ar est tel que défini précédemment et on obtient un composé de formule (2) que l'on 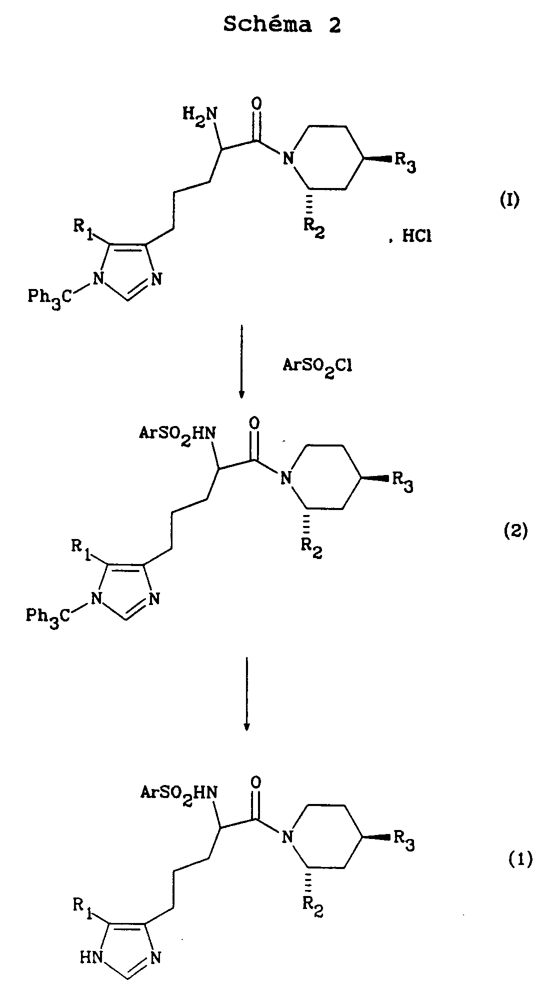
déprotège à chaud en présence d'acide acétique.

L'exemple A illustre cette synthèse.

### Exemple A

2*R*-[1(2*S*, 3*S*), 2α, 4β]]-1-[5-(1*H*-imidazol-4-yl)-2-[[(3-méthyl-1,2,3,4-tétrahydroquinoléin-8-yl)sulfonyl]amino]-1-oxopentyl]-4-méthylpipéridine-2-méthanol
A.1. acétate de [2*R*-[1(2*S*, 3*S*), 2α, 4β]]-[4-méthyl-1-[2-[[(3-méthyl-1,2,3,4-tétrahydroquinoléin-8-yl)sulfonyl]amino]-1-oxo-5-[1-(triphénylméthyl)-1*H*-imidazol-4-yl]pentyl]pipéridin-2-yl]méthyle
   A.1.1. chlorure de l'acide (*S*)-3-méthyl-1,2,3,4-tétrahydroquinoléine-8-sulfonique
      A.1.1.1. (*S*)-α-méthyl-2-nitrobenzènepropanoate de méthyle
         Sous atmosphère d'azote et sous agitation, on place 9,5 g (42 mmoles) de (*R*)-3-iodo-2-méthylpropanoate de méthyle dans 90 ml de benzène contenant 4,4 g du couple Zn(Cu) et 5,5 ml de diméthylacétamide. On agite pendant 15 minutes à la température ambiante puis on chauffe à 60 °C pendant 3 heures. Ensuite on laisse revenir le mélange à la température ambiante et on ajoute 1 g d'acétate de bis(tri-O-tolylphosphine) palladium en suspension dans 2 ml de benzène puis 7,5 g (30 mmoles) de 1-iodo-2-nitrobenzène en solution dans 20 ml de benzène. On chauffe, on laisse à 60 °C pendant 1 heure, on ajoute 100 ml d'acétate d'éthyle puis on filtre le milieu réactionnel sur célite. On lave le filtrat par 100 ml d'un solution 1 N d'acide chlorhydrique puis par 100 ml d'eau et on le sèche sur sulfate de magnésium. On purifie le résidu par chromatographie sur colonne de gel de silice en éluant par un mélange hexane/acétate d'éthyle (90/10).
         On obtient 3 g de produit sous forme d'une huile que l'on utilisera telle quelle dans l'étape suivante.
      A.1.1.2. (*S*)-3-méthyl-3,4-dihydroquinoléin-2(1*H*)-one
         *Méthode 1*
            Dans 40 ml de méthanol on place 3 g (14 mmoles) du produit obtenu dans l'étape précédente et on ajoute 100 mg d'oxyde de platine. On hydrogène sous une pression de 50 psi dans un appareil de Parr pendant 8 heures puis on filtre, on essore le catalyseur et on évapore le filtrat à sec. On récupère le résidu et on le triture dans l'éther. On le recristallise dans l'éther éthylique.
            On obtient 1,5 g de produit sous forme de cristaux Point de fusion = 117-119 °C
         *Méthode 2*
            Sous atmosphère d'azote et sous agitation, on place 31 g (135 mmoles) de (*R*)-3-iodo-2-méthylpropanoate de méthyle dans 310 ml de benzène contenant 19,75 g du couple Zn(Cu) et 20,3 ml de diméthylacétamide. On chauffe le mélange à 60 °C pendant 3 heures puis on le refroidit à la température ambiante. On ajoute 2,92 g d'acétate de bis(tri-O-tolylphosphine)palladium en une seule fois puis 19,75 g (90 mmoles) de 2-iodoaniline en solution dans 50 ml de benzène. On chauffe pendant 1 heure à 50 °C puis on laisse revenir le mélange à la température ambiante. On filtre sur célite, on lave par 2 fois 100 ml d'acétate d'éthyle et on rassemble les filtrats. On les lave successivement par 2 fois 100 ml d'une solution 1 N d'acide chlorhydrique, par 100 ml d'une solution saturée d'hydrogénocarbonate de sodium et par 50 ml d'une solution saturée de chlorure de sodium. On sèche sur sulfate de magnésium et on évapore à sec. On purifie le résidu par chromatographie sur colonne de gel de silice en éluant par du dichlorométhane.
            On obtient 4,9 g de produit.
            Point de fusion = 118-120 °C
      A.1.1.3. (*S*)-3-méthyl-1,2,3,4-tétrahydroquinoléine
         Dans 6 ml de tétrahydrofurane, on place 0,8 g (5 mmoles) du composé obtenu dans l'étape précédente et on ajoute, à 0 °C sous atmosphère d'azote, 17,5 ml d'une solution 1 M de borane dans le tétrahydrofurane. On chauffe à reflux pendant 1 heure et on ajoute lentement 5 ml d'eau. Ensuite on ajuste le pH du milieu réactionnel à 1 avec une solution 1 N d'acide chlorhydrique puis on chauffe au reflux pendant 2 heures. On laisse refroidir le mélange, on évapore, on reprend le résidu par 50 ml d'acétate d'éthyle et on le lave par 2 fois 50 ml d'une solution d'hydrogénocarbonate de sodium. On le sèche sur sulfate de magnésium et on évapore à sec. On purifie le résidu ainsi obtenu par chromatographie sur colonne de gel de silice en éluant par du dichlorométhane.
         On obtient 0,6 g de produit sous forme d'une huile que l'on utilisera telle quelle dans l'étape suivante.
         [α]_{D} + 79 ° (c = 3, méthanol)
      A.1.1.4. (*S*)-5-méthyl-5,6-dihydro-2*H*,4*H*-thiazolo[5,4,3-*ij*] quinoléin-2-one
         Sous atmosphère d'azote et sous agitation, on place 40,5 ml de toluène puis 2,28 ml (27 mmoles) de chlorure de chlorocarbonylsulfényle. On refroidit le milieu réactionnel à -50 °C puis on ajoute lentement un mélange de 3,5 g (23 mmoles) du composé obtenu dans l'étape précédente et de 3,24 g (28 mmoles) de *N*,*N*-diméthylbenzèneamine en solution dans 130 ml de toluène. On laisse le mélange remonter à la température ambiante, on ajoute 100 ml de toluène et on chauffe à 80 °C pendant 3 heures. On laisse refroidir et on lave par 2 fois 100 ml d'une solution 1 N d'acide chlorhydrique puis par 100 ml d'une solution saturée de NaCl. On sèche sur sulfate de magnésium puis on évapore à sec. On purifie le résidu ainsi obtenu par chromatographie sur colonne de gel de silice en éluant par du dichlorométhane.
         On obtient 4,3 g de produit.
         Point de fusion = 54-56 °C
      A.1.1.5. (*S*)-8,8'-dithiobis(3-méthyl-1,2,3,4-tétrahydroquinoléine)
         On reprend 1,4 g (6,8 mmoles) du composé obtenu dans l'étape précédente dans 14 ml de potasse alcoolique 3 N et on chauffe au reflux pendant 6 heures. On verse le milieu réactionnel sur 50 ml d'eau et on ajuste le pH du mélange à 4-5 avec de l'acide chlorhydrique. On extrait le mélange avec de l'éther, on recueille la phase éthérée, on la sèche sur sulfate de magnésium et on évapore à sec. On reprend le résidu dans 50 ml de benzène et on ajoute 10 g d'alumine. On agite à l'air pendant 18 heures, on filtre l'alumine et on la lave avec du dichlorométhane. On réunit les filtrats et on les évapore à sec. On purifie le résidu par chromatographie sur colonne de gel de silice en éluant par un mélange cyclohexane:acétate d'éthyle (95:5).
         On obtient 0,8 g de produit jaune.
         Point de fusion = 78-80 °C
      A.1.1.6. acide (*S*)-3-méthyl-1,2,3,4-tétrahydroquinoléine-8-sulfonique
         On dissout 0,8 g (4,5 mmoles) du composé obtenu dans l'étape précédente dans 4,51 g d'une solution d'acide sulfurique à 95 %, on place le milieu réactionnel dans un bain de glace et on ajoute lentement 1,49 ml d'eau oxygénée à 35 %. Ensuite on ajoute au milieu réactionnel de l'eau puis de la glace et on filtre le précipité ainsi obtenu. On le lave successivement avec 5 ml d'eau glacée, 3 fois 20 ml d'éther puis 10 ml de méthanol glacé. Finalement on rince à l'éther et on sèche à l'étuve sous pression réduite.
         On obtient 0,5 g de produit.
         Point de fusion = 255 °C (décomposition)
         [α]_{D}= + 38 ° [c = 0,2; méthanol:eau (50:50)]
      A.1.1.7. chlorure de l'acide (*S*)-3-méthyl-1,2,3,4-tétrahydroquinoléine-8-sulfonique
         On dissout 1,07 g de triphénylphosphine dans 7,5 ml de dichlorométhane à 0 °C. Sous atmosphère d'azote, à 0 °C, on ajoute goutte à goutte 0,3 ml de chlorure de sulfuryle puis on laisse le mélange remonter à la température ambiante. On ajoute ensuite lentement une solution contenant 0,47 g (2,1 mmoles) de l'acide obtenu dans l'étape précédente, 0,3 ml de triéthylamine et 12 ml de dichlorométhane. On agite pendant 1 heure à la température ambiante puis on verse le milieu réactionnel sur 200 ml de pentane. On le filtre, on évapore le filtrat à sec, on reprend le résidu dans 100 ml de pentane et on évapore à sec.
         On obtient 0,4 g de produit utilisé tel quel dans l'étape suivante.
   A.1.2. acétate de [2*R*-[1(2*S*, 3*S*), 2α, 4β]]-[4-méthyl-1-[2-[[(3-méthyl-1,2,3,4-tétrahydroquinoléin-8-yl) sulfonyl]amino]-1-oxo-5-[1-(triphénylméthyl)-1*H*-imidazol-4-yl]pentyl]pipéridin-2-yl]méthyle
      A 0,6 g (0,9 mmoles) du composé obtenu dans l'exemple 1, à 0 °C, on ajoute 0,4 g (2 mmoles) de chlorure de l'acide (*S*)-3-méthyl-1,2,3,4-tétrahydroquinoléine-8-sufonique obtenu selon la méthode décrite précédemment en solution dans 10 ml de dichlorométhane et 0,38 ml de triéthylamine dans 10 ml de dichlorométhane. On laisse sous agitation pendant une nuit à la température ambiante puis on lave successivement le mélange par 10 ml d'une solution 0,2 N d'acide chlorhydrique, 10 ml d'une solution saturée d'hydrogénocarbonate de sodium et 10 ml d'une solution saturée de chlorure de sodium. On filtre le milieu réactionnel, on le sèche sur sulfate de magnésium et on évapore à sec. On purifie le résidu par chromatographie sur colonne de gel de silice en éluant par un mélange dichlorométhane:éthanol (96:4).
      On obtient 0,96 g de produit.
      Point de fusion = 49 °C.
A.2. acétate de [2*R*-[1(2*S*, 3*S*), 2α, 4β]]-1-[5-(1*H*-imidazol-4-yl)-2-[[(3-méthyl-1,2,3,4-tétrahydroquinoléin-8-yl) sulfonyl]amino]-1-oxopentyl]-4-méthylpipéridine-2-yl] méthyle
   On place 0,65 g (0,74 mmoles) du composé obtenu dans l'étape précédente dans 10 ml d'acide acétique. On ajoute 2 ml d'eau et 8 ml de tétrahydrofurane puis on chauffe à 80 °C pendant 1 heure. On évapore le milieu réactionnel à sec et on purifie le résidu par chromatographie sur colonne de gel de silice en éluant par un mélange dichlorométhane:éthanol (90:10).
   On obtient 0,4 g de produit pur.
   Point de fusion = 47 °C
A.3. 2*R*-[1(2*S*, 3*S*), 2α, 4β]]-1-[5-(1*H*-imidazol-4-yl)-2-[[(3-méthyl-1,2,3,4-tétrahydroquinoléin-8-yl)sulfonyl]amino]-1-oxopentyl]-4-méthylpipéridine-2-méthanol
   On place 0,4 g (0,75 mmoles) du composé obtenu dans l'étape précédente dans 2 ml de méthanol, on refroidit à 0 °C et on ajoute goutte à goutte 1,5 ml d'une solution 1 N de soude. On laisse sous agitation pendant 2 heures à 0 °C, on évapore le méthanol et on suspend le résidu dans un mélange dichlorométhane:eau (50:50). On récupère la phase organique que l'on sèche sur sulfate de magnésium et que l'on évapore à sec. On reprend le résidu dans une solution 1 N d'acide chlorhydrique et on le purifie par chromatographie sur colonne de gel de silice en éluant par gradient d'acide chlorhydrique 0,01 N et d'acétonitrile (0 à 100 % d'acétonitrile). On rassemble les fractions contenant le produit et on les lyophilise.
   On obtient 0,3 g de produit.
   Point de fusion = 90 °C
   [α]_{D} = + 110 ° (c = 0,2; méthanol)

Les composés de formule (1) ainsi obtenus présentent des propriétés antithrombotiques et sont décrits dans la demande de brevet européen EP 0565396 de la demanderesse.

## Revendications

1. Composés répondant à la formule (I) 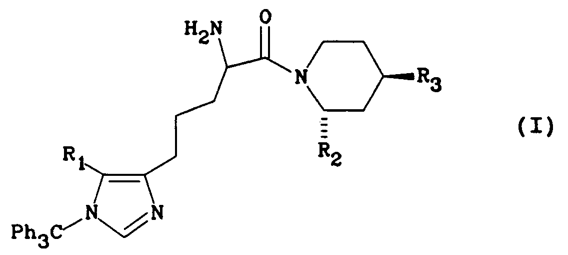 dans laquelle
R₁ représente soit un atome d'hydrogène, soit un groupe (C₁-C₄) alkyle,
R₂ représente soit un atome d'hydrogène, soit un groupe (C₁-C₄)alcoxycarbonyle, soit un groupe carboxylique, soit un groupe carboxylate de sodium, soit un groupe -CH₂OR₄ où R₄ est un atome d'hydrogène, un groupe (C₁-C₄)alkyle ou (C₁-C₄)acyle, soit un groupe -CONR₅R₆, soit un groupe -CN₄R₅ où R₅ est un atome d'hydrogène ou un groupe (C₁-C₄)alkyle et R₆ est un atome d'hydrogène, un groupe (C₁-C₄)alkyle ou hydroxy ou (C₁-C₄)alcoxy ou (C₁-C₃)alcoxyphényle et
R₃ représente un groupe (C₁-C₄)alkyle,
ainsi que leurs sels d'addition aux acides.

2. Composés selon la revendication 1 caractérisés en ce que
R₁ représente soit un atome d'hydrogène, soit un groupe (C₁-C₄) alkyle,
R₂ représente soit un groupe carboxylique, soit un groupe éthoxycarbonyle, soit un groupe hydroxycarboxamide, soit un groupe hydroxyméthyle, soit un groupe 1*H*-tétrazolyle et
R₃ représente un groupe méthyle ou éthyle.

3. Composés selon l'une quelconque des revendications 1 et 2 caractérisés en ce que la configuration préférentielle du groupe pipéridinyle est [2R,4R] et la configuration préférentielle du carbone asymétrique de la partie acide aminé centrale 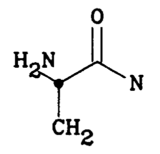 est [S].

4. Procédé de préparation des composés selon la revendication 1, procédé caractérisé en ce que l'on déprotège un composé de formule (V) 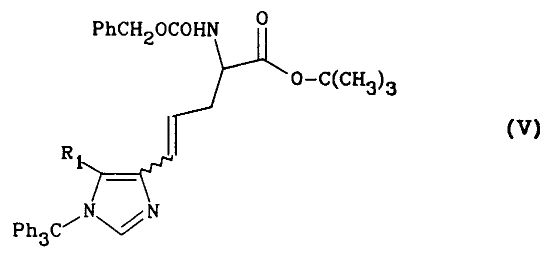 dans laquelle R₁ est tel que défini dans la revendication 1 pour obtenir un composé de formule (VI) 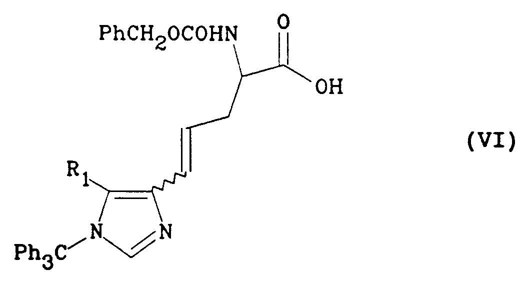 que l'on fait réagir avec une pipéridine de formule (VII) 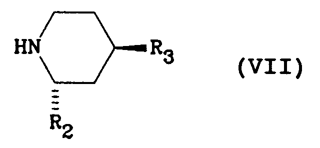 dans laquelle R₂ et R₃ sont tels que définis dans la revendication 1 et on obtient un composé de formule (VIII) 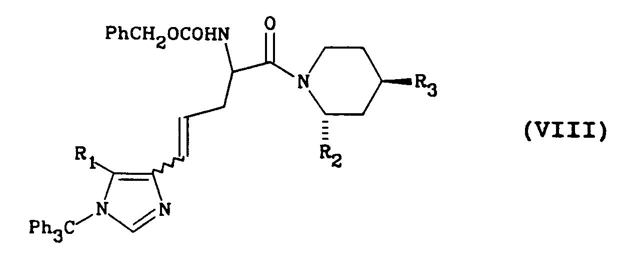 que l'on soumet à une hydrogénation catalytique.

5. Utilisation des composés selon l'une quelconque des revendications 1 à 3 pour la synthèse de composés de formule (1) 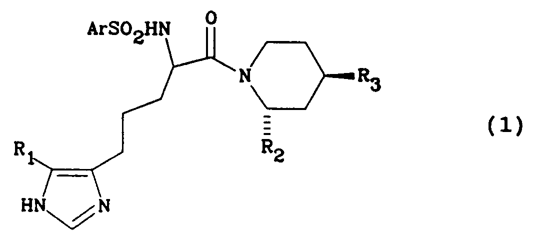 dans laquelle
R₁, R₂ et R₃ sont tels que définis dans la revendication 1 et Ar représente soit un groupe naphtalén-1-yle substitué par un groupe di(C₁-C₄)alkylamino, soit un groupe 6,7-di(C₁-C₄)alcoxy naphtalén-1-yle, soit un groupe quinoléin-8-yle substitué en 3 par un groupe (C₁-C₄)alkyle, soit un groupe 1,2,3,4-tétrahydroquinoléin-8-yle substitué en 3 par un groupe (C₁-C₄) alkyle, soit un groupe 1*H*-indazol-7-yle.
